# EUROPEAN PATENT SPECIFICATION

(11) **EP 3 743 202 B1**
(45) Date of publication and mention of the grant of the patent: **18.11.2026**
(21) Application number: 19714847.1
(22) Date of filing: 28.01.2019
(51) Int. Cl.: B01J 8/06, B01J 8/00, B01J 8/02, C07C 1/02, C07C 7/00, C07C 7/144, B01D 53/22, C07C 41/01, C07C 29/152, B01J 19/18

(54) **PROCESS FOR THE HYDROGENATION OF CARBON DIOXIDE**
VERFAHREN ZUR HYDRIERUNG VON KOHLENDIOXID
PROCÉDÉ D'HYDROGÉNATION DU DIOXYDE DE CARBONE

(30) Priority: 26.01.2018 EP 18153753
(43) Date of publication of application: 02.12.2020
(73) Proprietor: Nederlandse Organisatie voor toegepast- natuurwetenschappelijk onderzoek TNO, 2595 DA 's-Gravenhage (NL)
(72) Inventor: GOETHEER, Earl Lawrence Vincent, 2595 DA 's-Gravenhage (NL); LINDERS, Marco Johannes Gerardus, 2595 DA 's-Gravenhage (NL); GARCIA MORETZ-SOHN MONTEIRO, Juliana, 2595 DA 's-Gravenhage (NL); BHARDWAJ, Rajat, 2595 DA 's-Gravenhage (NL)
(74) Representative: V.O.
(86) International application number: PCT/NL2019/050046
(87) International publication number: WO 2019/147132

(56) References cited:
- CA-A1- 2 186 222
- US-A- 3 358 750
- US-A1- 2004 064 002
- US-A1- 2009 170 964
- US-B1- 8 425 854
- US-B2- 8 921 619
- CHAN BUN ET AL: "Design of Effective Zeolite Catalysts for the Complete Hydrogenation of CO 2", JOURNAL OF THE AMERICAN CHEMICAL SOCIETY, vol. 128, no. 16, 1 April 2006 (2006-04-01), pages 5322 - 5323, XP093015931, ISSN: 0002-7863, DOI: 10.1021/ja0602492
- DE FALCO MARCELLO ET AL: "Selective membrane application for the industrial one-step DME production process fed by CO2 rich streams: Modeling and simulation", INTERNATIONAL JOURNAL OF HYDROGEN ENERGY, vol. 42, no. 10, 1 March 2017 (2017-03-01), AMSTERDAM, NL, pages 6771 - 6786, XP093146804, ISSN: 0360-3199, DOI: 10.1016/j.ijhydene.2017.02.047
- WANG PENG ET AL: "Recent advances in membrane distillation processes: Membrane development, configuration design and application exploring", JOURNAL OF MEMBRANE SCIENCE, vol. 474, 1 January 2015 (2015-01-01), NL, pages 39 - 56, XP093146802, ISSN: 0376-7388, DOI: 10.1016/j.memsci.2014.09.016
- GALLUCCI F ET AL: "An experimental study of CO2 hydrogenation into methanol involving a zeolite membrane reactor", CHEMICAL ENGINEERING AND PROCESSING, ELSEVIER SEQUOIA, LAUSANNE, CH, vol. 43, 13 December 2003 (2003-12-13), pages 1029 - 1036, XP002546298, ISSN: 0255-2701, [retrieved on 20031213], DOI: 10.1016/J.CEP.2003.10.005
- BOS J: "A novel condensation reactor for efficient CO2 to methanol conversion for storage of renewable electric energy - ScienceDirect", 15 October 2015 (2015-10-15), XP055596550, Retrieved from the Internet <URL:https://www.sciencedirect.com/science/article/pii/S1385894714013916?via=ihub> [retrieved on 20190614]
- HAUT B ET AL: "Development and analysis of a multifunctional reactor for equilibrium reactions: benzene hydrogenation and methanol synthesis", CHEMICAL ENGINEERING AND PROCESSING, ELSEVIER SEQUOIA, LAUSANNE, CH, vol. 43, no. 8, 13 December 2004 (2004-12-13), pages 979 - 986, XP002733680, ISSN: 0255-2701, [retrieved on 20031213], DOI: 10.1016/J.CEP.2003.09.006
- ROHDE M P ET AL: "Fischer-Tropsch synthesis with in situ H"2O removal - Directions of membrane development", MICROPOROUS AND MESOPOROUS MATERIALS, ELSEVIER, AMSTERDAM, NL, vol. 115, no. 1-2, 15 February 2008 (2008-02-15), pages 123 - 136, XP023521070, ISSN: 1387-1811, [retrieved on 20080215], DOI: 10.1016/J.MICROMESO.2007.10.052
- NAZELY DIBAN ET AL: "Membrane Reactors for in Situ Water Removal: A Review of Applications", INDUSTRIAL & ENGINEERING CHEMISTRY RESEARCH, vol. 52, no. 31, 7 August 2013 (2013-08-07), pages 10342 - 10354, XP055596518, ISSN: 0888-5885, DOI: 10.1021/ie3029625

## Description

The invention is in the field of chemical reactors and chemical processes. In particular, the invention is directed to a process for the hydrogenation of carbon dioxide into methanol and/or dimethyl ether.

The process of hydrogenating carbon dioxide into methanol and/or dimethyl ether (also referred to as DME) can *i.a.* be used to lower CO₂ concentration in the atmosphere. In addition, the process can also be used to further valorize biogas and to provide a broader applicability of said biogas. For instance, by separating biogas into CO₂ and methane, the methane can be reformed into hydrogen (H₂) and the obtained hydrogen can be allowed to react with the CO₂ to form methanol and/or DME. This concept is not limited to biogas since in principle any CO₂-containing gas can be valorized and provided with a broader applicability by hydrogenating the CO₂ therein.

The hydrogenation of CO₂ is believed to involve the following reactions:

CO₂ + 3H₂ 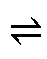 CH₃OH + H₂O (I)

CO₂ + H₂ 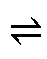 CO + H₂O (II)

CO + 2H₂ 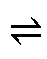 CH₃OH (III)

2 CH₃OH 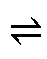 CH₃OCH₃ + H₂O (IV)

The reaction CO₂ to MeOH can proceed directly (equation I), or via the intermediate CO (equation II and III). Depending on the process parameters, the obtained MeOH can react further into DME (equation IV). As is apparent from equations I-IV, water is produced as a side product concomitantly with MeOH and/or DME. In addition, since these reactions (I-IV) are in equilibrium under the process conditions, full conversion to the products can in principle not be obtained under thermodynamic equilibrium conditions. Therefore, in order to maximize the conversion into methanol and/or DME, it is preferred that at least one of the reaction products which is formed in the reaction (*i.e.* water, methanol and/or DME) is removed from the reaction medium.

Examples of conventional methods for the removal of the reaction products include condensation in a recycle configuration, reactive adsorption of water, and *in-situ* water-consuming reaction. US 2004/064002 discloses water vapor permeation through a membrane to remove the water from a reaction of MeOH to DME. In WO2015/030578, a reactor and a process are described comprising two stages including a zone or stage wherein a liquid condensate of methanol and water is condensed. Bos et al., Chemical Engineering Journal 278 (2015) 527-532 discloses a condensation reactor for CO₂ to methanol conversion for storage of renewable electric energy. Haut et al., Chemical Engineering and Processing 43 (2004) 979-986 discloses the development and analysis of a multifunctional reactor for benzene hydrogenation and methanol synthesis. CA2186222 disclose the synthesis of methanol and proposes shifting the equilibrium of the reaction in the direction of the product(s) by removing at least one product from the reaction mixture by membrane permeation. Further, De Falco et al., International Journal of Hydrogen Energy 42, 10, 9 March 2017, Pages 6771-6786, discloses the modeling ans assessment of the integration of a microporous zeolite membrane, highly selective to steam water in a one-step DME synthesis reactor. However, all these methods are associated with several drawbacks.

For instance, a recycle configuration results in a bulky process design with installation of separate equipment, piping and control, while the use of solid system for the adsorption or reactive-consumption of water pose challenging regeneration and handling, also making the overall process bulky and expensive. Furthermore, a solid adsorption system involves regeneration which leads to a discontinuous type of operation and higher temperatures are needed for regeneration. Although the use of water vapor permeation through a membrane is elegant, this conventional method is associated with concomitant removal of H₂ from the reaction and decrease of the overall conversion. The combined condensation of methanol and water as disclosed in WO2015/0305578 requires a subsequent separation of the water and methanol in order to obtain dry methanol and results in a liquid methanol product, whereas it may be preferred to maintain a gaseous methanol product if is going to sequentially be converted into products in a gaseous phase reaction. This process is therefore relative high in energy consumption.

It is an object of the present invention to provide a process for the hydrogenation of carbon dioxide, that addresses at least one of these drawbacks.

A further object of the present invention is to provide an improved hydrogenation of carbon dioxide, by enhancing reaction rates towards the production of methanol and/or DME, improving catalyst lifetime, improving reactor design and intensification of process of production of methanol and/or DME from several steps to single reaction-separation-removal unit, and/or by providing a conversion beyond equilibrium constraints due to selective removal of at least one reaction product from the reaction mixture.

The present inventors found that these objects can at least partially be met by a combination of permeation of the formed water through a membrane and condensation of said water after permeation. This provides a method to selectively remove the water from the process, without the requirement of bulky process design with installation of separate equipment. The permeation and condensation are carried out in the same reactor, which reactor is another aspect of the present invention (*vide infra*)*.* In particular, this method enables the *in-situ* removal of condensed water from the reaction within the same reactor in which the hydrogenation reaction is carried out.

Accordingly, the present invention is directed to a process for the hydrogenation of carbon dioxide, wherein said process comprises reacting carbon dioxide with hydrogen to form methanol and/or dimethyl ether, and water as a side product, and wherein said process further comprises removing said water from the process by a combination of permeation of said water through a membrane and condensation of the water.

The process of the the present invention is carried out in a membrane reactor for the hydrogenation of carbon dioxide, said membrane reactor comprising a reaction compartment comprising a catalyst bed, a permeate compartment, and a water-permeable membrane that separates the reaction compartment and the permeate compartment. Said permeate compartment comprises a condensing surface such that the reactor is particularly suitable for carrying out the method of the present invention. The condensing surface in the permeate compartment is configured and is connected to a means for cooling (herein also referred to as cooling means) such that during operation of the reactor, the water can condense on the surface. Said reactor further comprises
- an inner wall (5) that is tubular bounding an inner space that defines the reaction compartment (2);
- an outer wall (7) that is tubular and that is at least partially co-axially arranged around said inner wall, wherein said outer wall (7) and inner wall (5) bound an outer space that defines the permeate compartment (4); and
wherein said inner wall (5) comprises the water-permeable membrane (51), and wherein said condensing surface is connected to the outer wall (7);
or said reactor comprises
- an inner wall (5) that is tubular bounding an inner space that defines the permeate compartment (4);
- an outer wall (7) that is tubular and that is at least partially co-axially arranged around said inner wall, wherein said outer wall (7) and inner wall (5) bound an outer space that defines the reaction compartment (2); and

wherein said inner wall (5) comprises the water-permeable membrane (51) and wherein the condensing surface is located away from the inner wall,
and wherein the means for cooling comprises an active-cooling device that comprises a passage through which a cooling liquid can flow.

The process comprises:
- reacting carbon dioxide with hydrogen to form methanol and/or dimethyl ether, and water as a side product,
- active cooling of the condensing surface by flowing a cooling liquid through the passage; and
- removing said water from the process by a combination of permeation of said water through the water-permeable membrane and condensation of the water on the condensing surface after permeation.

The means for cooling may be such to effect active and passive cooling. Examples of passive cooling include heat sinks and heat conductive materials that can be passively cooled by conductive cooling due to a temperature difference. Active cooling however is typically more effective.

Active cooling is obtained by providing the means for cooling with an active-cooling device that comprises a tube through which a cooling liquid flows Suitable cooling liquids include e.g. water. A combination of active and passive cooling, e.g. by providing both liquid cooling through the tube and a heat sink can also be used.

The water-permeable membrane is typically not exclusively permeable for water. For instance, hydrogen molecules, which are also present in the reaction compartment, are smaller than water molecules and may also (albeit undesirably) permeate the membrane. In fact, due to this permeation of hydrogen, substantial amounts of hydrogen removal from the reaction mixture have been observed in the aforementioned conventional methods. It is believed that the requirement of a sweeping gas stream to remove the vapor, also removes the permeated hydrogen. Advantageously, the presence of the condensing surface mitigates the requirement of the sweeping gas stream and the condensing surface selectively condensates water while hydrogen remains gaseous under the condensation and reaction conditions. With this method, separation of water from the reaction mixture and from H₂, may be carried out in one step, limiting the need of additional equipment for individual steps of separation and purification.

Typical reaction conditions include elevated temperatures and pressures. In particular, reacting carbon dioxide with hydrogen may be carried out at a temperature in the range of 150-400 °C, preferably in the range of 200 to 300 °C, more preferably about 250 °C and/or at a pressure in the range of 1-10 MPa, preferably in the range of 2-8 MPa, more preferably about 5 MPa.

The water-permeable membrane (herein also referred to as the membrane) can accordingly suitably function at these reaction conditions. Therefore, the water-permeable membrane is preferably a hydrophilic membrane, which preferably comprises a zeolite membrane, an amorphous membrane or a polymer membrane. Particularly suitable zeolite membranes comprise mordenite (MOR), ZSM-5, chabazite (CHA), silicalite-1 (SIL-1), Z4A, faujasite (FAU), Si/Al variant MFI, and the like. Suitable amorphous membranes may comprise Al₂O₃/SiO₂. The polymer membranes may comprise ceramic-supported polymers (CSP), as these are particularly suitable for functioning at elevated temperatures. Membranes that are used for the water removal in Fischer-Tropsch processes may also be suitably used for the present invention (see *e.g.* Rohde et al. Microporous and Mesoporous Materials 115 (2008) 123-136).

The catalyst bed may comprise any known catalyst that suitably catalyzes the hydrogenation of carbon dioxide (see *e.g.* Gallucci et al. Chemical Engineering and Processing 43 (2004) 1029-1036 and references therein). Particularly suitable catalysts comprise copper, zinc oxide, zirconia, palladium, cerium(IV) oxide or combinations thereof. The catalyst may be supported on suitable supports such as alumina or silica. Advantageously, the present invention can increase the lifetime of the catalyst due to an increased removal of the water. As such, less water may condense in the catalyst particles, which can increase both performance and lifetime.

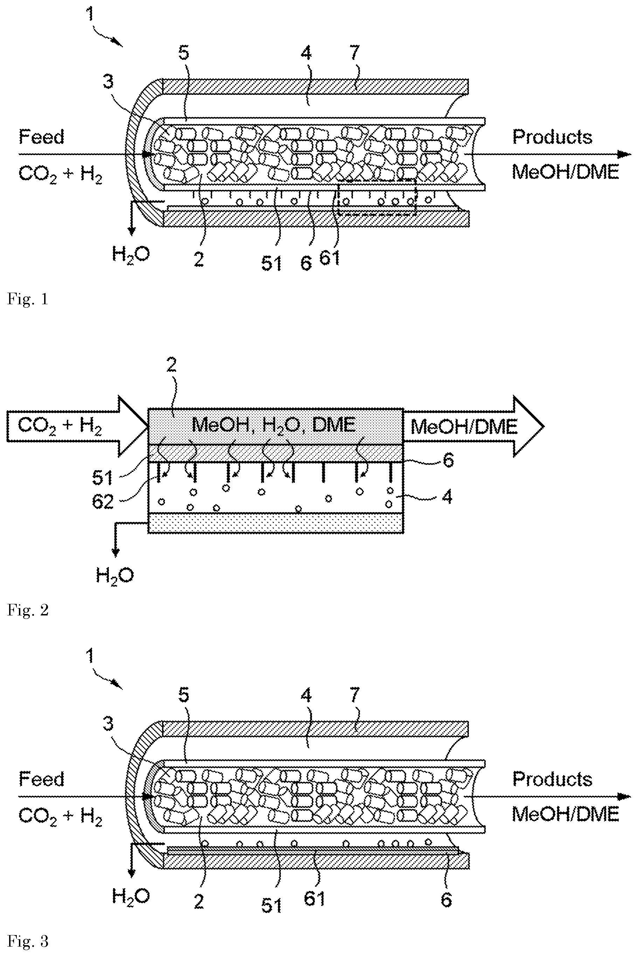

In figure 1, a reactor not in accordance with the present invention is illustrated. The reactor comprises:
- an inner wall (5) bounding an inner space that defines the reaction compartment (2);
- an outer wall (7) that is arranged around said inner wall, wherein said outer wall (7) and inner wall (5) bound an outer space that defines the permeate compartment (4);
wherein said inner wall (5) comprises the water-permeable membrane (51). As illustrated in figure 1, the inner wall and outer wall are tubular and the outer wall is co-axially arranged around said inner wall.

In a preferred embodiment, the condensing surface is part of a protruding surface elements (62) that are connected to or part of the cooling means. In a particular embodiment, the protruding surface elements are connected to said inner wall (5) and protruding into the permeate compartment. An example of this embodiment, presented in a manner that is not in accordance with the invention, is illustrated in figure 2. In an alternative, embodiment, the protruding surface is connected to outer wall (7) and protruding into the permeate compartment (not shown). This may be preferred as condensation of water in the membrane is preferably prevented because condensed water in the membrane can block the membrane' pores. The protruding surface elements and thus the surface can have various shapes. For instance, the surface may be undulating or jagged to increase the total surface area. Suitable shapes may result from protruding elements such as rods, bars, blades and the like. Such protruding elements may comprise at least part of the active-cooling device such a tubing.

In particular embodiments not according to the invention, the condensing surface is situated in the reactor in the vicinity of the membrane. As such, the permeated water can travel a relatively short path before it condenses. The inventors found however, that in accordance with the invention, it is actually preferred that the cooling means and the condensing surface is situated in the reactor away from the membrane. For the hydrogenation reaction, typical reaction temperatures are around 250 °C due to which the membrane may have a temperature of about 200 °C, while it is preferred that the condensing surface is kept at a much lower temperature (*vide infra*). Accordingly, such a temperature difference can more easily and efficiently be maintained when the cooling means and condensing surface are situated away from the membrane, or at least by situating the cooling means and condensing surface disconnected from the membrane and the inner wall. In addition, it can preferred condensed water in the membrane's pores. Away from the inner wall and in the vicinity of the outer wall herein means that the cooling means are closes to the outer wall than to the inner wall.

In figure 3, an embodiment of the reactor is shown, wherein the configuration is similar to that as illustrated in figure 1, but the means for cooling (6) in this embodiment is situated away from the inner wall (5) and more in the vicinity of the outer wall (7).

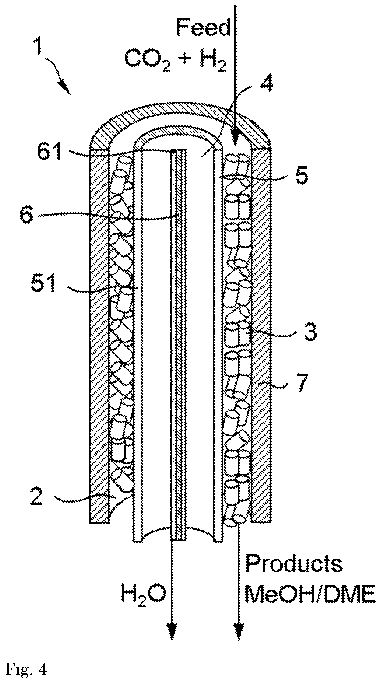

In figure 4, yet another configuration of the membrane reactor is illustrated. In this particular embodiment, which is preferred, the membrane reactor (1) comprises
- an inner wall (5) bounding an inner space that defines the permeate compartment (4);
- an outer wall (7) that is arranged around said inner wall, wherein said outer wall (7) and inner wall (5) bound an outer space that defines the reaction compartment (2);
wherein said inner wall (5) comprises the water-permeable membrane (51). Thus, the embodiment wherein the permeate compartment is at least partially enclosed by reaction compartment, is an inverse configuration of the reactor illustrated in figures 1 and 3 wherein the reactor compartment is at least partially enclosed by the permeate compartment. As further illustrated in figure 4, preferably the cooling means (6) and the condensing surface (61) are located away from the inner wall. For instance, in an co-axial, tubular configuration of the reactor, the cooling means can be placed in the (longitudinal) axis of the reactor, as illustrated in figure 4 as well. The cooling means however, can have various shapes, and are not confined to a straight configuration as illustrated in figure 4. For instance, the cooling means can be a U-shaped, helical shaped, jagged and/or undulated tube, a various thereof. The cooling means can also comprise the protruding elements such as rods, bars, blades and the like.

The condensing surface is actively cooled, meaning that it is maintained at a temperature by which water can condensate under the, generally elevated, reaction pressures. The condensing surface is cooled actively by providing a cooling liquid stream, for instance (relatively) cool water, within tubing near the condensing surface. It is to be understood that the cooling liquid should preferably be prevented from contacting the reactants in the reactor, such a hydrogen. The cooling liquid therefore is typically separated from the reaction compartment and the permeate compartment by a wall. For effective condensation under the reaction conditions, the condensing surface is preferably maintained at a temperature in the range of 50 to 150 °C. The inventors however found that water can particularly effectively be condensed at less than 100 °C, preferably less than 50 °C.

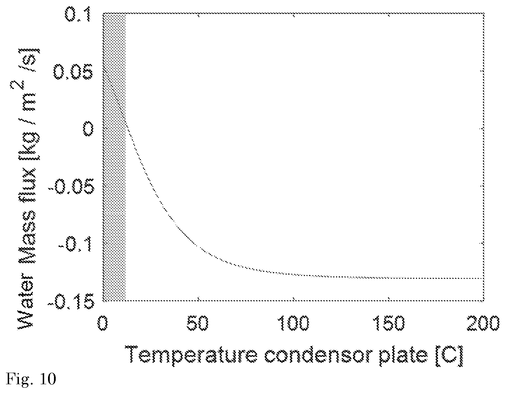

Surprisingly, the inventors found that in case the condensing surface is 10 °C or less, condensation of the water occurs in such an effective manner, that a water mass flux across the membrane can be achieved (see Figure 10). The water mass flux across the membrane results in even better DME yields. For this reason, it is preferred that the cooling means of the reactor described herein, are adapted to be able to cool, preferably actively cool, the condensing surface to a temperature of less then 100 °C, preferably less than 50 °C, most preferably less than 10 °C, during operation of the reactor wherein a reaction is carried out at a temperature in the range of 150-400 °C, preferably in the range of 200 to 300 °C, more preferably about 250 °C.

The condensed water can be collected and let out the permeate compartment.

For the purpose of clarity and a concise description features are described herein as part of the same or separate embodiments, however, it will be appreciated that the scope of the invention may include embodiments having combinations of all or some of the features described.

The invention can be illustrated by the following non-limiting examples.

### Example 1 - Effect of water removal on DME production

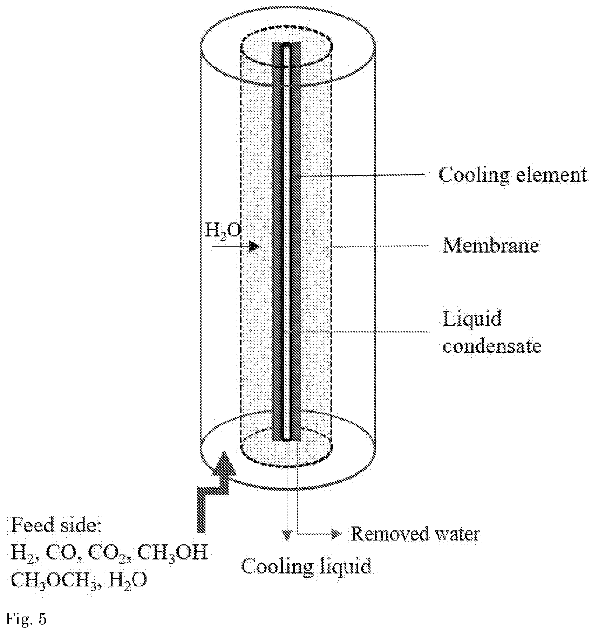

The following reaction and reactor is analysed *in silico.* In a membrane reactor as illustrated in Figure 5, at the feed side (*i.e.* in the reaction compartment) a carbon dioxide hydrogenation reaction takes place to produce methanol and subsequent conversion to dimethyl ether, at 250 °C and 50 bar, according to the reactions 1-4. Reaction 3 is the combination of reaction 1 and 2. By removing water from reaction 4, the reaction can be shifted to the right-hand side to produce more dimethyl ether. Therefore, *in-situ* removal of water from the mixture of H₂, H₂O, CO, CH₃OH, CH₃OCH₃ will drive the reactions towards more DME production. To show the effect of water removal, two models were created.
(1) *CO* + 2 *H*₂ ↔ *CH*₃*OH*
(2) *CO*₂ *+ H*₂ ↔ *CO + H*₂*O*
(3) *CO*₂ + 3H₂ ↔ *CH*₃*OH* + H₂O
(4) *2 CH*₃*OH* ↔ CH₃OCH₃ *+ H*₂*O*

In the first model kinetic equations and equilibrium constants for reaction 1-3 were used from Portha et al., Erena et al. and Alharbi et al., to show the establishment of chemical equilibrium in DME production. (see also Portha et al., Ind. Eng. Chem. Res., 56 (2017) 13133-13145, Ereña et al., Chem. Eng. J. 174 (2011) 660-667 and Alharbi et al. ACS Catal 5 (2015) 7186-7193. This equilibrium limits the amount of DME produced. By modelling the chemical equilibrium with and without water removal, it is shown that *in-situ* water removal with membrane reactor leads to an increase in DME production. Table 1 contains the starting conditions and pressures for the equilibrium model.

**Table 1: Input values for equilibrium model**

| **Input values:** | |
|---|---|
| T [°C] | 250 |
| P [bar] | 50 |
| Start pressure CO₂ [bar] | 7.5 |
| Start pressure CO [bar] | 7.5 |
| Start pressure H₂ [bar] | 35 |

A second model calculates the steady state water removal by the membrane reactor, driven by the pressure difference between feed and permeate side. The calculated water removal from the feed side is used as an input for the first model in the graph, to show the increase in DME production. Additionally, variations in air gap and temperature of the cooling element show the effect of different parameter on the membrane reactor's performance

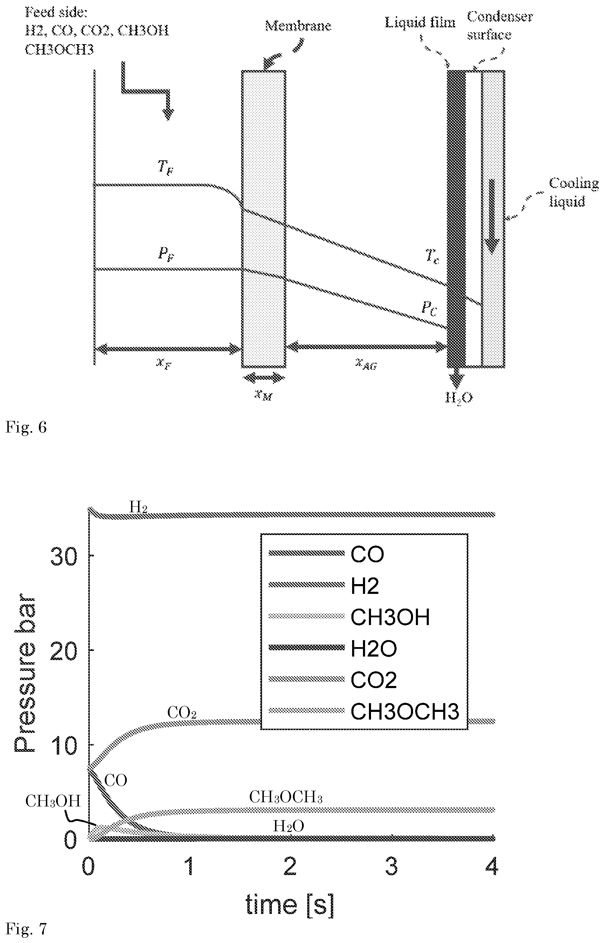

For modelling a steady state in-situ water removal during conversion from CO₂ to dimethyl ether (DME), theory was used that is commonly applied for air gap membrane distillation processes used in water treatment. Figure 6 shows the temperature and pressure profiles for the membrane reactor from Figure 5. Water removal through the membrane is driven by a pressure difference between the water gas pressure on the feed side *P_{F}* and the water pressure just above the liquid film on the condenser surface *P_{C}*.

The water gas pressure on the feed side is calculated by multiplying its calculated vapor fraction with the total pressure on the feed side of the reaction. ${P}_{F} = {y}_{i} ∗ {P}_{total}$ (5)

On the other side of the membrane, the vapor pressure just above the liquid film on the condenser surface is described by the Antoine equation (see equation 6). ${P}_{C} = {10}^{4.6543 - \frac{1435.264}{{T}_{cool} - 64.848} \left(1.0E+5\right)}$ (6)

The dominant mechanism for the water vapor mass flux is indicated by the Knudsen number. Equation 7 is used to calculate the Knudsen number, with *k_{B},*T,r, *d*_{*H*2*O*} and P as Boltzmann constant, temperature, membrane thickness, diameter water molecule and pressure. ${K}_{n} = \frac{{k}_{B} T}{2 {Lπd}_{H 2 O}^{2} P √ 2}$ (7)

A membrane thickness of 1 mm gives a Knudsen number smaller than 0.01 indicating that molecular diffusion through the air gap will be the mass transfer limiting step at 250 °C and 50 bar. Molecular diffusion through the air gap is usually the limitation in mass transfer in air gap membrane distillation.

Equation 8 gives the water flux for transition flow. Based on membrane properties in Table 2, a flux of 0.097 *kg*/*m²*/*s* was calculated. $J = \frac{εPDM Δ p}{\left(τδ+b\right) RT \left({P}_{a}\right)}$ (8)

**Table 2: properties used in flux calculations**

| **Properties** | **Values used in calculation** |
|---|---|
| Temperature condenser surface [°C ] | 8 |
| Radius condenser element [mm] | 1 |
| Air gap [mm] | 2 |
| Vapor fraction H₂O in feed gases yi | 2.94E-4 |
| Length membrane [m] | 0.1 |
| Membrane thickness [mm] | 1 |
| Membrane pore radius r [µm] | 1 |
| Porosity *ε* | 0.56 |
| Tortuosity *τ* | 2 |
| Diffusion coefficient water-air [m²/s] | 2.8E-5 |
| Flow rate H₂O feed side [l/s] | 3 |

Equation 8 represents the one-dimensional flux, whereas the experimental setup is cylindrical. As a simplification, the surface of the cylindrical membrane area was multiplied with the one-dimensional flux to get the total flux to be deposited on the inner cylindrical cooling element.

The result of the *in silico* reaction are illustrated in Figures 7 to 9.

Figure 7 shows the model for establishing chemical equilibrium for reactions 1-4. This represents the chemical reactions that occur on the feed side of the membrane reactor, without water removal, starting from the pressures in Table 1. This can be compared to the situation where the cooling element at the permeate side of the membrane reactor is at the same temperature as the feed side and there is no *in-situ* water removal. Water is produced in reaction 3, but consumed in the water gas shift reaction (reaction 2 to the left hand side). Overall, without water removal, the end concentration of DME is 6.21 vol%.

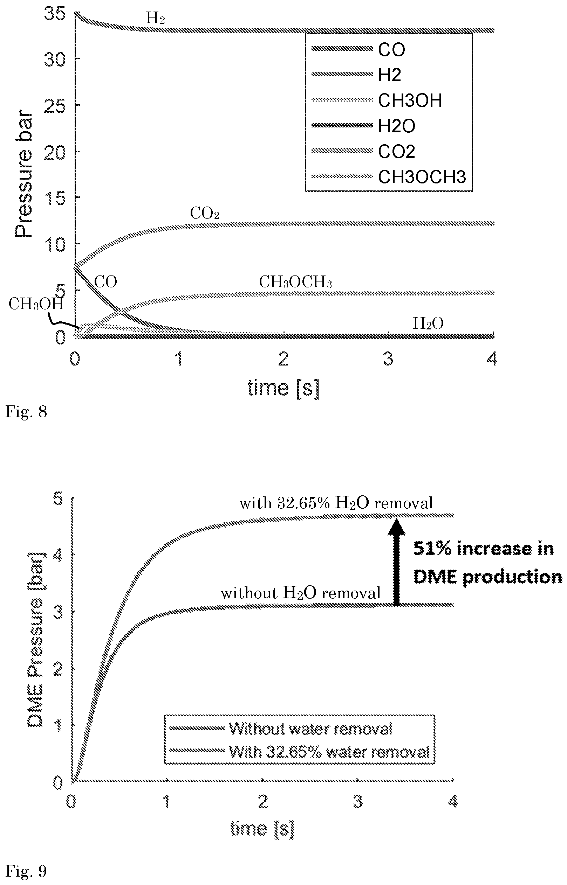

Figure 8 shows the same chemical equilibrium model with a constant water removal where 33% of the created water is removed. This amount of water removal matches with the water flux calculated in the model of the in-situ water removal membrane reactor with the standard parameters in Table 2. An equilibrium model with an added constant water removal corresponds to the situation where the reactions 2 and 3 shift more to the right-hand side of the equations due to the water removal, giving a higher DME yield. With water removal matching to the flux of water removal calculated in the membrane reactor model, the yield of DME rises with 51 vol%. Figure 9 shows the 51 vol% increase in DME yield due to water removal.

### Example 2 - Effect of condensing surface temperature on the flux of water across the membrane

Based on the models described in Example 1, the water mass flux over the membrane can be calculated, depending on the temperature of the condensing surface. The results are depicted in Figure 10, from which it can be deduced that at condensing surface temperatures of >50 °C, essentially no water flux is expected. The operating temperatures of membrane is close to 200 °C at which the condensation on/ inside the membranes is not taking place. The results further show that active cooling with condensing temperature of <10 °C results in positive flux for water flux across the membrane.

## Claims

1. Process for the production of methanol and/or dimethyl ether by hydrogenation of carbon dioxide carried out in a membrane reactor (1) for the hydrogenation of carbon dioxide, said membrane reactor comprising:
- a reaction compartment (2) comprising a catalyst bed (3),
- a permeate compartment (4) and
- a water-permeable membrane (51) separating the reaction compartment and the permeate compartment, wherein said permeate compartment comprises a condensing surface (61) and a means for cooling (6) that is connected to said condensing surface (61) such that during operation of the reactor, water can condense on the condensing surface;
wherein said reactor comprises
- an inner wall (5) that is tubular bounding an inner space that defines the reaction compartment (2);
- an outer wall (7) that is tubular and that is at least partially co-axially arranged around said inner wall, wherein said outer wall (7) and inner wall (5) bound an outer space that defines the permeate compartment (4); and
wherein said inner wall (5) comprises the water-permeable membrane (51), and wherein said condensing surface is connected to the outer wall (7), or
wherein said reactor comprises
- an inner wall (5) that is tubular bounding an inner space that defines the permeate compartment (4);
- an outer wall (7) that is tubular and that is at least partially co-axially arranged around said inner wall, wherein said outer wall (7) and inner wall (5) bound an outer space that defines the reaction compartment (2); and
wherein said inner wall (5) comprises the water-permeable membrane (51) and wherein the condensing surface is located away from the inner wall,
and wherein the means for cooling comprises an active-cooling device that comprises a tube through which a cooling liquid flows,
wherein said process comprises:
- reacting carbon dioxide with hydrogen to form methanol and/or dimethyl ether, and water as a side product,
- active cooling of the condensing surface by flowing a cooling liquid through the tube;
- removing said water from the process by a combination of permeation of said water through the water-permeable membrane and condensation of the water on the condensing surface after permeation, and
wherein said water is removed from the process without a sweeping gas stream.

2. Process in accordance with claim 1, wherein the condensing surface comprises protruding surface elements (62).

3. Process in accordance with any of the previous claims, wherein said water-permeable membrane comprises a hydrophilic membrane, preferably comprising a zeolite membrane, an amorphous membrane or a polymer membrane.

4. Process in accordance with any of the previous claims, wherein said catalyst bed comprises copper, zinc oxide, zirconia, palladium, cerium(IV) oxide or combinations thereof.

5. Process in accordance with any of the previous claims, wherein the condensing surface has a temperature of less than 150 °C, preferably less than 100 °C, more preferably less than 50 °C, even more preferably less than 10 °C.

6. Process in accordance with any of the previous claims, wherein reacting carbon dioxide with hydrogen is carried out at a temperature in the range of 150-400 °C, preferably in the range of 200-300 °C, more preferably about 250 °C and/or at a pressure in the range of 1-10 MPa, preferably in the range of 2-8 MPa, more preferably about 5 MPa.

7. Process in accordance with any of the previous claims, wherein said carbon dioxide and/or said hydrogen originate from biogas.

## Patentansprüche

1. Verfahren zur Herstellung von Methanol und/oder Dimethylether durch Hydrierung von Kohlendioxid, durchgeführt in einem Membranreaktor (1) zur Hydrierung von Kohlendioxid, der Membranreaktor umfassend:
- einen Reaktionsraum (2), umfassend ein Katalysatorbett (3),
- ein Permeatkompartiment (4) und
- eine wasserdurchlässige Membran (51), den Reaktionsraum und den Permeatraum trennend, wobei der Permeatraum eine Kondensationsfläche (61) und ein Mittel zum Kühlen (6) umfasst, das so mit der Kondensationsfläche (61) verbunden ist, dass während des Betriebs des Reaktors Wasser auf der Kondensationsfläche kondensieren kann;
wobei der Reaktor umfasst
- eine Innenwand (5), die rohrförmig einen Innenraum begrenzt, der den Reaktionsraum (2) definiert;
- eine Außenwand (7), die rohrförmig ist und zumindest teilweise koaxial um die Innenwand angeordnet ist, wobei die Außenwand (7) und die Innenwand (5) einen Außenraum begrenzen, der das Permeatkompartiment (4) definiert; und
wobei die Innenwand (5) die wasserdurchlässige Membran (51) umfasst, und wobei die Kondensationsfläche mit der Außenwand (7) verbunden ist, oder
wobei der Reaktor umfasst
- eine Innenwand (5), die rohrförmig einen Innenraum begrenzt, der das Permeatkompartiment (4) definiert;
- eine Außenwand (7), die rohrförmig ist und zumindest teilweise koaxial um die Innenwand angeordnet ist, wobei die Außenwand (7) und die Innenwand (5) einen Außenraum begrenzen, der den Reaktionsraum (2) definiert; und
wobei die Innenwand (5) die wasserdurchlässige Membran (51) umfasst und wobei die Kondensationsfläche von der Innenwand entfernt angeordnet ist,
und wobei das Mittel zum Kühlen eine Aktivkühlvorrichtung umfasst, die ein Rohr umfasst, durch das eine Kühlflüssigkeit strömt,
wobei das Verfahren umfasst:
- Reagieren von Kohlendioxid mit Wasserstoff unter Bildung von Methanol und/oder Dimethylether und Wasser als Nebenprodukt,
- aktives Kühlen der Kondensationsfläche durch Strömenlassen einer Kühlflüssigkeit durch das Rohr;
- Entfernen des Wassers aus dem Verfahren durch eine Kombination von Permeation des Wassers durch die wasserdurchlässige Membran und Kondensation des Wassers auf der Kondensationsfläche nach Permeation, und wobei das Wasser aus dem Verfahren entfernt wird ohne einen Spülgasstrom.

2. Verfahren nach Anspruch 1, wobei die Kondensationsfläche vorstehende Oberflächenelemente (62) umfasst.

3. Verfahren nach einem der vorhergehenden Ansprüche, wobei die wasserdurchlässige Membran eine hydrophile Membran umfasst, vorzugsweise umfassend eine Zeolithmembran, eine amorphe Membran oder eine Polymermembran.

4. Verfahren nach einem der vorhergehenden Ansprüche, wobei das Katalysatorbett Kupfer, Zinkoxid, Zirconiumdioxid, Palladium, Cerium(IV)-oxid oder Kombinationen davon umfasst.

5. Verfahren nach einem der vorhergehenden Ansprüche, wobei die Kondensationsfläche eine Temperatur von weniger als 150 °C, vorzugsweise weniger als 100 °C, bevorzugter weniger als 50 °C, noch bevorzugter weniger als 10 °C hat.

6. Verfahren nach einem der vorhergehenden Ansprüche, wobei das Reagieren von Kohlendioxid mit Wasserstoff durchgeführt wird bei einer Temperatur im Bereich von 150-400 °C, vorzugsweise im Bereich von 200-300 °C, bevorzugter etwa 250 °C, und/oder bei einem Druck im Bereich von 1-10 MPa, vorzugsweise im Bereich von 2-8 MPa, bevorzugter etwa 5 MPa.

7. Verfahren nach einem der vorhergehenden Ansprüche, wobei das Kohlendioxid und/oder der Wasserstoff aus Biogas stammen.

## Revendications

1. Procédé pour la production de méthanol et/ou de diméthyléther par hydrogénation de dioxyde de carbone mise en œuvre dans un réacteur à membrane (1) pour l'hydrogénation de dioxyde de carbone, ledit réacteur à membrane comprenant :
- un compartiment de réaction (2) comprenant un lit de catalyseur (3),
- un compartiment de perméat (4) et
- une membrane perméable à l'eau (51) séparant le compartiment de réaction et le compartiment de perméat,
dans lequel ledit compartiment de perméat comprend une surface de condensation (61) et un moyen de refroidissement (6) qui est connecté à ladite surface de condensation (61) de façon que, lors du fonctionnement du réacteur, de l'eau puisse se condenser sur la surface de condensation ;
dans lequel ledit réacteur comprend
- une paroi intérieure (5) qui est tubulaire et délimite un espace intérieur qui définit le compartiment de réaction (2) ;
- une paroi extérieure (7) qui est tubulaire et qui est disposée au moins partiellement de manière coaxiale autour de ladite paroi intérieure,
dans lequel lesdites paroi extérieure (7) et paroi intérieure (5) délimitent un espace extérieur qui définit le compartiment de perméat (4) ; et
dans lequel ladite paroi intérieure (5) comprend la membrane perméable à l'eau (51), et dans lequel ladite surface de condensation est connectée à la paroi extérieure (7), ou
dans lequel ledit réacteur comprend
- une paroi intérieure (5) qui est tubulaire et délimite un espace intérieur qui définit le compartiment de perméat (4) ;
- une paroi extérieure (7) qui est tubulaire et qui est disposée au moins partiellement de manière coaxiale autour de ladite paroi intérieure,
dans lequel lesdites paroi extérieure (7) et paroi intérieure (5) délimitent un espace extérieur qui définit le compartiment de réaction (2) ; et
dans lequel ladite paroi intérieure (5) comprend la membrane perméable à l'eau (51), et dans lequel ladite surface de condensation est située à distance de la paroi intérieure,
et dans lequel le moyen de refroidissement comprend un dispositif de refroidissement actif qui comprend un tube à travers lequel s'écoule un liquide de refroidissement,
lequel procédé comprend :
- la réaction de dioxyde de carbone avec de l'hydrogène pour former du méthanol et/ou du diméthyléther, et de l'eau en tant que sousproduit,
- le refroidissement actif de la surface de condensation par circulation d'un liquide de refroidissement à travers le tube ;
- l'élimination de ladite eau du procédé par une combinaison de perméation de ladite eau à travers la membrane perméable à l'eau et de condensation de l'eau sur la surface de condensation après perméation, et
dans lequel ladite eau est éliminée du procédé sans courant de gaz de balayage.

2. Procédé selon la revendication 1, dans lequel la surface de condensation comprend des éléments de surface protubérants (62).

3. Procédé selon l'une quelconque des revendications précédentes, dans lequel ladite membrane perméable à l'eau comprend une membrane hydrophile, comprenant de préférence une membrane de zéolite, une membrane amorphe ou une membrane polymère.

4. Procédé selon l'une quelconque des revendications précédentes, dans lequel ledit lit de catalyseur comprend du cuivre, de l'oxyde de zinc, de la zircone, du palladium, de l'oxyde de cérium(IV) ou des combinaisons de ceux-ci.

5. Procédé selon l'une quelconque des revendications précédentes, dans lequel la surface de condensation a une température inférieure à 150 °C, de préférence inférieure à 100 °C, mieux encore inférieure à 50 °C, plus particulièrement inférieure à 10 °C.

6. Procédé selon l'une quelconque des revendications précédentes, dans lequel la réaction de dioxyde de carbone avec de l'hydrogène est mise en œuvre à une température située dans la plage allant de 150 à 400 °C, de préférence située dans la plage allant de 200 à 300 °C, mieux encore d'environ 250 °C et/ou sous une pression située dans la plage allant de 1 à 10 MPa, de préférence située dans la plage allant de 2 à 8 MPa, et mieux encore d'environ 5 MPa.

7. Procédé selon l'une quelconque des revendications précédentes, dans lequel ledit dioxyde de carbone et/ou ledit hydrogène sont issus de biogaz.
